# EUROPEAN PATENT APPLICATION

(11) **EP 1 013 244 A1**
(43) Date of publication of application: **28.06.2000**
(21) Application number: 99310208.6
(22) Date of filing: 16.12.1999
(51) Int. Cl.: A61F 2/36

(54) **Prosthetic femoral component**

(30) Priority: 21.12.1998 GB 9828210
(71) Applicant: BENOIST GIRARD ET CIE, 14201 Hérouville-Saint-Clair Cedex (FR)
(72) Inventor: Ling, Robin Sydney Mackwood, Dartmouth, Devon T06 0HB (GB); Gie, Graham Allan, Yeoford, Devon EX17 5EZ (GB); Timperley, Andrew John, St. Leonard's, Exeter EX2 4PA (GB); Storer, John Andrew, 14250 Jouaye Mondaye, Bayeux (FR)
(74) Representative: Bridge-Butler, Alan James

(57) **Abstract**

A femoral component (10) of a replacement hip joint having a tapered collarless stem (11) for fixing in a medullary canal by cement and in which, at the proximal end (12) of the stem (11), there is a pronounced laterally projecting heel (15) adapted to extend into the greater trochanter of the femur into which it is to be fitted.

## Description

This invention relates to a femoral component of a replacement hip joint of the "Exeter" type which has a tapered collarless stem for fixing in a medullary canal by cement.

The "Exeter" type femoral component of the kind shown in British Patent No 1 409 054 is well known and comprises a neck which carries a ball head for cooperation with an acetabular socket. The neck is connected to a tapered collarless stem. Thus there is no collar for resting either on the bone or the cement in the area where the stem joins the neck of the implant. This type of stem has evolved so that the stem can be aiven a polished finish to help it slide down inside the bone cement and the present invention relates to this type of femoral component.

It has been found that it is preferable to provide anchoraae for the stem to resist as much as possible rotation of the stem after implantation.

The present invention is intended to provide a femoral component which takes advantage of this facility.

In known constructions of femoral components of this type, for example as shown in British Patent No 1 409 054, the lateral side of the component is usually straight or taDered in relation to its longitudinal axis and leads directly to a shoulder at the proximal end which curves around to meet the neck which carries the ball head. Such components are fitted by reaming out the medullary canal appropriately. There is, however, space in bone within the greater trochanter and the femoral component according to the present invention is intended to take advantage of this to provide greater resistance to stem rotation after implantation.

According to the present invention a femoral component of a reDlacement hip joint which has a tapered collarless stem for fixing in a medullary canal by cement and in which, at the proximal end of the stem, there is a pronounced laterally projecting heel adapted to extend into the greater trochanter of the femur into which it is to be fitted.

Thus, the distal facinq part of the heel can provide a lateral portion which is greater than that of known collarless stems and which is particularly adapted to resist torque.

It will be appreciated that, in order to fit the femoral component according to the invention, it is necessary to broach the medullary canal so that there is a cavity within the greater trochanter.

The invention can be performed in various ways, but one embodiment will now be described by way of example and with reference to the accompanying drawing, in which:
Figure 1 is a side view a femoral component according to the invention shown in position in a femur; and
Figure 2 is an end view of the device shown in Figure 1.

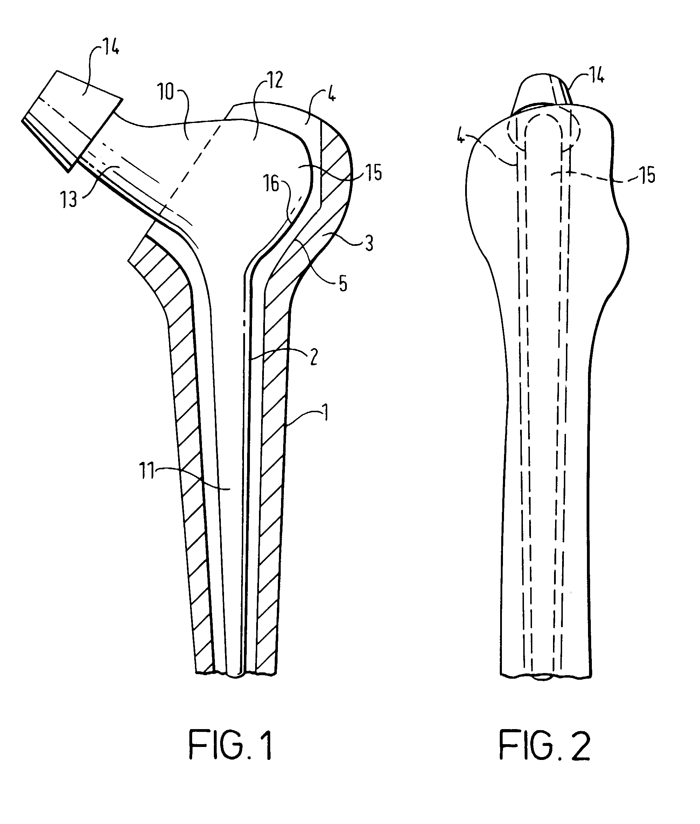

As shown in Figure 1, a cross-sectioned femur is indicated by reference numeral 1. The intramedullary canal 2 of the femur has been broached to accept a femoral component, and it will be seen that, at the greater trochanter indicated by reference numeral 3, the canal 2 has been opened out in a lateral direction which causes an exposed portion of the canal 4 to extend as a slot within the greater trochanter 3.

The proximal Dart of the prepared canal has a pronounced angled portion 5.

The especially adapted femoral component for use in such a canal is indicated by reference numeral 10 and comprises a stem 11, a proximal Dortion 12 and a neck 13 on which is located a spigot 14 to accept a ball head (not shown).

The proximal end of the stem, where it leads into the proximal portion 12, has a pronounced laterally projecting heel 15 which extends into the greater trochanter 3 when it is fitted. As will be seen from Figure 1, the particular widely-splayed shape of the proximal portion 12 provides a distal surface 16 to the heel, which can be concave, convex or straight, but increases the lateral width of the component to provide not only a greater area of contact to absorb loads and resist torsional displacement, but to absorb loads in general.

The invention has great advantages over components of standard shape, and can be fitted in most Datients, provided there is sufficient healthy bone.

## Claims

1. A femoral component of a replacement hip joint having a tapered collarless stem for fixina in a medullary canal by cement and in which, at the proximal end of the stem, there is a pronounced laterally projecting heel adapted to extend into the greater trochanter of the femur into which it is to be fitted.

2. A femoral component as claimed in claim 1 in which the distal surface of said heel is substantially concave.

3. A femoral component as claimed in claim 1 in which the distal surface of said heel is substantially convex.

4. A femoral component as claimed in claim 1 in which the distal surface of said heel is substantially straight.
